# EUROPEAN PATENT SPECIFICATION

(11) **EP 2 177 181 B1**
(45) Date of publication and mention of the grant of the patent: **17.06.2015**
(21) Application number: 09275090.0
(22) Date of filing: 05.10.2009
(51) Int. Cl.: A61F 2/90

(54) **Wavily deformable stent and method for producing the same**
Wellenförmig verformbarer Stent und Verfahren zu dessen Herstellung
Endoprothèse déformable de manière ondulée et son procédé de fabrication

(30) Priority: 16.10.2008 KR 20080101638
(43) Date of publication of application: 21.04.2010
(73) Proprietor: Taewoong Medical Co., Ltd., Kimpo-si Kyonggi do 415-873 (KR); Shin, Kyong-Min, Seodaemun-ku Seoul (KR)
(72) Inventor: Shin, Kyong-Min, Seodaemun-ku, Seoul (KR); Myung, Byung-Cheol, Deukyang-ku, Goyang-si Kyongki-do (KR)
(74) Representative: McCartney, Jonathan William

(56) References cited:
- WO-A-01/35864
- WO-A-03/057079
- DE-U1-202008 014 828
- US-A1- 2007 179 590

## Description

### Field of the Invention

The present invention relates to a stent for use in expanding the stenosed part of a bile duct or other bodily organs generated by a cancer or other causes. More specifically, the present invention pertains to a wavily deformable stent that can be fixed inside a stenosed part in a wavily deformed state with no likelihood of unwanted displacement and a method for producing a wavily deformable stent. The wavily deformable stent of the present invention is produced by interlacing wires in different intervals from part to part along a longitudinal direction to form a hollow cylindrical net body having a plurality of alternating high-rigidity and low-rigidity sections.

### Background of the Invention

In general, a medical stent has been used to expand the stenosed part of a bile duct or other bodily organs generated by a cancer or other causes.

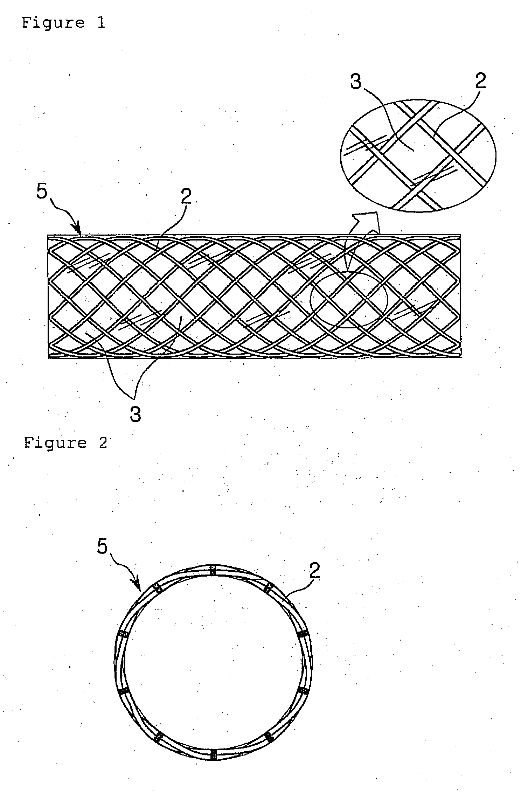

As shown in Figs. 1 and 2, the conventional stent is produced by interlacing super-elastic shape-memory alloy wires or stainless steel wires 2 into a hollow cylindrical net body 5 of specified length with a multiplicity of rhombic meshes 3.

WO 03/057079 A1 describes a body insertable tubular stent that includes discrete tubular segments in an alternating sequence of segments having high axial stiffness and segments having low axial stiffness. The lower axial stiffness segments are intended for placement along more severely curved regions of the body vessel, to provide a greater degree of stent conformity to the vessel. The more axially flexible segments can be provided by winding a metallic or polymeric strand at a higher pitch along such segments, thus to form a higher braid angle in a braided stent. In such cases the more axially flexible segments also exert a higher radial force when the stent is radially compressed. Alternatively, the stent can consist of multiple interbraided strands, with each strand incorporating a higher number of filaments (at least two) along axially stiff segments, and incorporating a lower number of filaments (at least one) along axially flexible segments. In another alternative version, the device is formed of one or more resilient strands, each strand including a biostable filament and a bioabsorbable filament, so that after implantation the radial force and axial stiffness gradually decrease in vivo.

As an alternative example, there has been provided a stent of the type including a cylindrical net body and a sleeve-like film arranged inside or outside the net body, the film being made of polytetrafluoroethylene (PTFE) or silicon.

The stent is designed to have a diameter slightly larger than that of a bile duct or other bodily organs to be surgically treated and a length a little greater than that of a stenosed part.

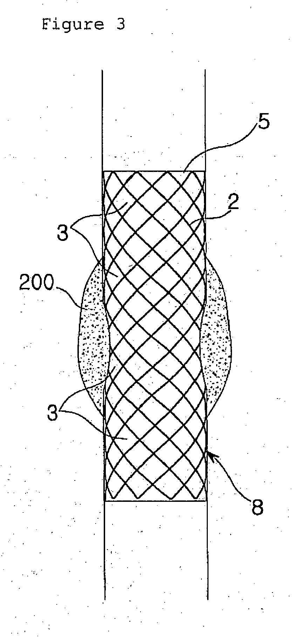

As illustrated in Fig. 3, the net body 5 of the stent 8 serves to expand the stenosed part 200 with the elastic expansion force thereof, thereby widening the tract or cavity of a bodily organ. The stent thus installed is kept in place by the contact force acting between itself and the stenosed part of the bodily organ.

Inasmuch as the net body 5 of the stent 8 makes contact with the stenosed part with a uniform contact force over the whole length thereof, the stent may be slidingly moved out of the original place over time by various kinds of causes. That is to say, there is a problem in that the stent is displaced from the stenosed part.

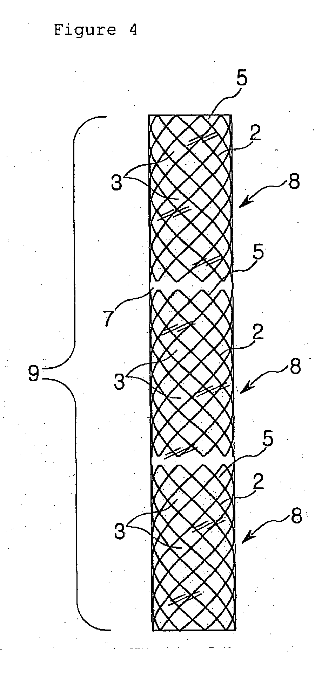

As a solution to this problem, there has been provided a connection-type stent 9. As shown in Fig. 4, the connection-type stent 9 includes a plurality of cylindrical net segments 8 arranged in an end-to-end relationship with one another and a unitary film 7 for covering the net segments 8 to interconnect them, the unitary film 7 being made of polytetrafluoroethylene (PTFE) or silicon.

In the connection-type stent 9, the respective net segments 8 serve to expand a stenosed part in different positions with the elastic expansion force thereof, thereby widening the tract or cavity of a bodily organ. The portions of the stent 9 with no net segment, namely the portions of the stent 9 consisting of only the film 7, are unable to fully expand the stenosed part. Thus, the connection-type stent 9 is fixed to the stenosed part in a wavily deformed state with an increased contact force. This helps prevent the stent 9 from being displaced out of the stenosed part during its use.

With the connection-type stent 9 mentioned above, however, the film 7 may be gradually dissolved over time by a secreting fluid or a bodily fluid flowing through or existing in a bile duct or other bodily organs. As a result, the net segments 8 are separated away from one another and sometimes displaced out of the stenosed part. The net segments 8 thus separated may hinder the flow of the bodily fluid or may move to other places, causing disorders to other organs. In this case, a surgical operation needs to be performed in order to remove the net segments 8.

In addition, there is known a stent including a cylindrical net body and an engaging protrusion formed on the outer circumferential surface of the net body for engagement with the inner wall surface of an organ. The engaging protrusion is formed to protrude radially outwards by interlacing an independent wire. The stent of this type poses a problem in that it may cause damage to the organ.

### Summary of the Invention

In view of the above-noted and other problems inherent in the prior art, it is an object of the present invention to provide a wavily deformable stent that can be firmly situated in a stenosed part of a bodily organ with little or no likelihood of displacement, and a method for producing the wavily deformable stent.

In accordance with one aspect of the present invention, there is provided a wavily deformable stent comprising a hollow cylindrical net body formed of elastically deformable wires interlaced with each other, wherein the net body extends in a longitudinal direction and terminates at open opposite ends, wherein the net body includes at least one high-rigidity section and at least one low-rigidity section less rigid than the high-rigidity section, and wherein the or each high-rigidity section and the or each low-rigidity section are arranged continuously and alternately along the longitudinal direction; the low- and high-rigidity sections being formed of crossed and bent interlaced wires, the wires in the high-rigidity section being interlaced at a first, narrow interval and the wires in the low-rigidity section being interlaced at a second, broader interval.

The stent of the present invention may further include a resin film layer for covering one of the inner and outer circumferential surfaces of the net body, the film being made of polytetrafluoroethylene or silicon. The stent of the present invention may further include a pair of enlarged extension portions provided at the opposite ends of the net body, the enlarged extension portions being greater in diameter than the net body.

In accordance with another aspect of the present invention, there is provided a method for producing a wavily deformable stent, comprising the steps of: preparing elastically deformable wires; and interlacing the wires with each other by crossing and bending to form a hollow cylindrical net body having at least one high-rigidity section and at least one low-rigidity section less rigid than the high-rigidity section, wherein the net body extends in a longitudinal direction and terminates at open opposite ends and wherein the or each high-rigidity section and the or each low-rigidity section are arranged continuously and alternately along the longitudinal direction; wherein the at least one high-rigidity section is formed by crossing and bending the interlaced wires at a first, narrow interval; and the at least one low-rigidity section is formed by crossing and bending the interlaced wires at a second, broader interval.

With the stent of the present invention, the alternating high-rigidity and low-rigidity sections of the cylindrical net body can expand the stenosed part of a bodily organ with different forces and therefore can be situated inside the stenosed part in a wavily deformed state along the length of the stenosed part. This assists in increasing the contact force acting between the stenosed part and the stent, thereby preventing the stent from being displaced out of the stenosed part.

### Brief Description of the Drawings

The above and other objects and features of the present invention will become apparent from the following description of preferred embodiments, given in conjunction with the accompanying drawings, in which:
Figs. 1 and 2 are front and side views illustrating one example of conventional stents;
Fig. 3 is a view depicting the stent situated inside the stenosed part of a bodily organ;
Fig. 4 is a view illustrating another example of conventional stents;
Fig. 5 is a view showing a wavily deformable stent in accordance with one embodiment of the present invention;
Fig. 6 is a view showing a wavily deformable stent in accordance with another embodiment of the present invention, which stent is provided with a resin film layer;
Figs. 7 through 11 are views showing a wavily deformable stent in accordance with a further embodiment of the present invention, which stent is provided with enlarged extension portions;
Fig. 12 is a perspective view showing a jig used in producing the wavily deformable stent of the present invention; and
Fig. 13 is a view illustrating the wavily deformable stent of the present invention situated inside the stenosed part of a bodily organ.

### Detailed Description of the Preferred Embodiments

Preferred embodiments of the present invention will now be described in detail with reference to the accompanying drawings.

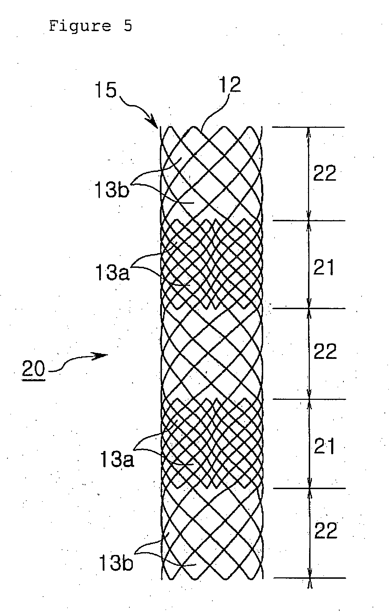

Referring first to Fig. 5, a wavily deformable stent 20 according to one embodiment of the present invention includes a hollow cylindrical net body 15 formed of elastically deformable wires 12 interlaced with each other. The wires 12 are made of, e.g., ultra elastic shape-memory alloy or stainless steel.

The net body 15 extends in a longitudinal direction and terminates at open opposite ends. In the illustrated embodiment, the net body 15 includes two high-rigidity sections 21 and three low-rigidity sections 22 less rigid than the high-rigidity section 21. The high-rigidity sections 21 and the low-rigidity sections 22 are arranged continuously and alternately along the longitudinal direction. Although the high-rigidity sections 21 and the low-rigidity sections 22 are formed in plural numbers in the illustrated embodiment, the present invention is not limited thereto. The number of the high-rigidity sections 21 and the low-rigidity sections 22 may be greater or lesser than illustrated.

In the high-rigidity sections 21, the wires 12 are interlaced at a narrow interval so that each of the high-rigidity sections 21 can have a plurality of first rhombic meshes 13a with a relatively small average size. In other words, the high-rigidity sections 21 are formed of the wires 12 interlaced at an increased density. Therefore, the high-rigidity sections 21 show relatively high rigidity.

In the low-rigidity sections 22, the wires 12 are interlaced at a broad interval so that each of the low-rigidity sections 22 can have a plurality of second rhombic meshes 13b greater in average size and smaller in number than the first meshes 13a of the high-rigidity sections 21. In other words, the low-rigidity sections 22 are formed of the wires 12 interlaced at a reduced density. Therefore, the low-rigidity sections 22 show rigidity smaller than that of the high-rigidity sections 21. This means that the high-rigidity sections 21 are less pliable than the low-rigidity sections 22 and therefore can support the stenosed part of a bodily organ with a greater expanding force.

In the illustrated embodiment, each of the high-rigidity sections 21 is shorter than each of the low-rigidity sections 22. Alternatively, the high-rigidity sections 21 and the low-rigidity sections 22 may differ in length from each other. The length of the high-rigidity sections 21 and the low-rigidity sections 22 may vary with the size of the stenosed part, the shape of the stenosed part, the kinds of the bodily organ and so forth. Likewise, the difference in rigidity between the high-rigidity sections 21 and the low-rigidity sections 22 may be set in many different ways depending on the characteristics of the stenosed part.

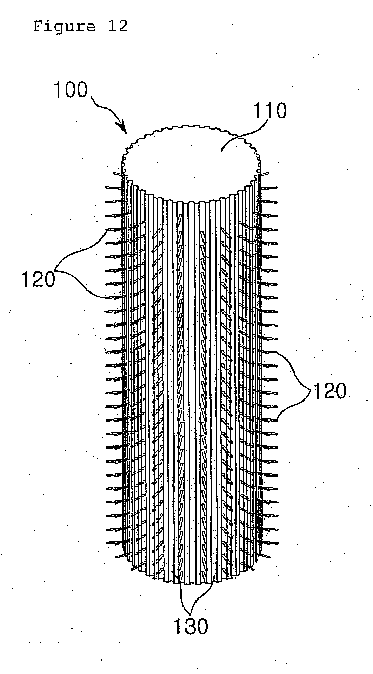

The net body 15 of the stent 20 can be produced through the use of a jig 100 shown in Fig. 12. The jig 100 includes a cylinder 110 and a plurality of pins 120 protruding radially outwards from the circumferential surface of the cylinder 100. The cylinder 100 has a plurality of longitudinal wire-insertion grooves 130 formed on the circumferential surface thereof at an equal spacing. The pins 120 are removably fixed at the intersecting points of longitudinal dividing lines and circumferential dividing lines, both of which are drawn on the circumferential surface of the cylinder 100 at an equal interval.

When forming the high-rigidity sections 21, the wires 12 are crossed and bent at a narrow interval through every neighboring row of the pins 120 to leave the first rhombic meshes 13a of small size between the crossed wires 12.

In contrast, when forming the low-rigidity sections 22, the wires 12 are crossed and bent at a wide interval through every other row of the pins 120 to leave the second rhombic meshes 13b of large size between the crossed wires 12.

By alternately repeating these crossing and bending operations, it is possible to produce the net body 15 along which high-rigidity sections 21 and the low-rigidity sections 22 are arranged continuously and alternately.

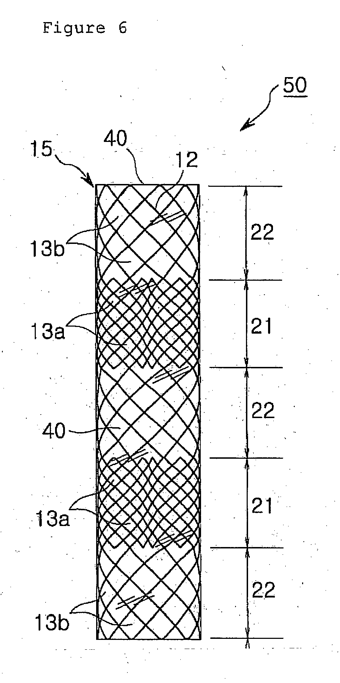

Referring to Fig. 6, there is shown a wavily deformable stent 50 in accordance with another embodiment of the present invention. The stent 50 of this embodiment is structurally the same as the stent 20 of the preceding embodiment, except that a resin film layer 40 is formed on the net body 15. The same component parts will be designated by like reference characters and will be omitted from description.

The resin film layer 40 is made of, e.g., polytetrafluoroethylene (PTFE) and silicon. The resin film layer 40 may be divided into a first resin film layer formed on the inner circumferential surface of the net body 15 and a second resin film layer formed on the outer circumferential surface of the net body 15. In this case, it is preferred that the first resin film layer and the second resin film layer are made of different resins. For example, if the first resin film layer is made of polytetrafluoroethylene, the second resin film layer will be made of silicon. Conversely, if the first resin film layer is made of silicon, the second resin film layer will be made of polytetrafluoroethylene.

The resin film layer 40 thus formed serves mainly to prevent the stenosed part of the bodily organ from growing into the stent 50 through the meshes 13a and 13b of the net body 15. In case where the first and second resin film layers made of different resins are formed on the inner and outer circumferential surfaces of the net body 15 as set forth above, it is possible to enhance the resistance of the resin film layers to the bodily fluid, thereby allowing the stent 50 to perform its function for a prolonged period of time.

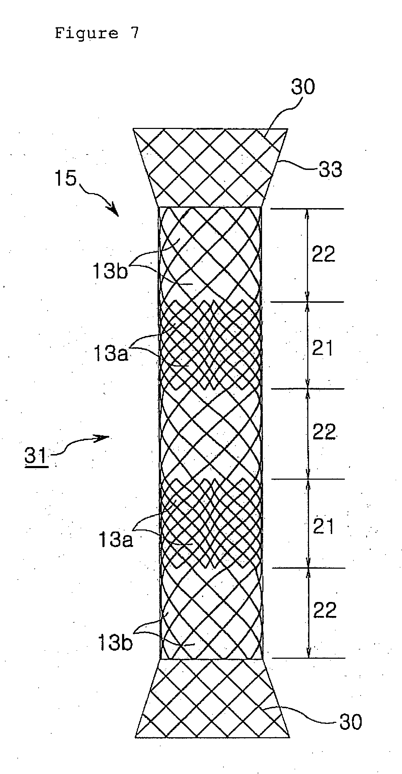

Referring to Figs. 7 through 11, there is shown a wavily deformable stent 31 or 60 in accordance with a further embodiment of the present invention. The stent 31 or 60 of this embodiment is structurally the same as the stent 20 or 50 of the preceding embodiment, except that a pair of enlarged extension portions 30 is provided at the opposite ends of the net body 15. The same component parts will be designated by like reference characters and will be omitted from description.

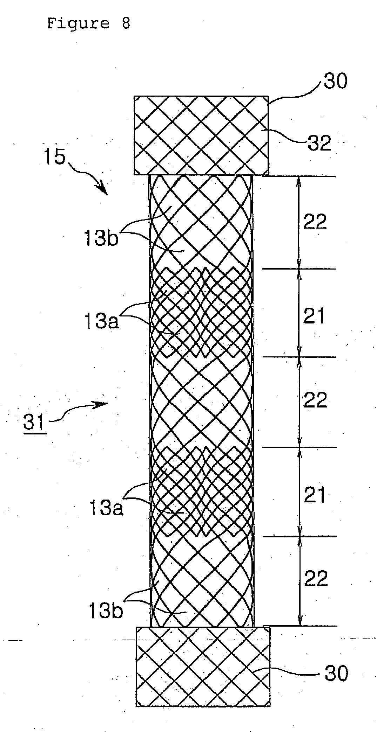

The stent 31 shown in Fig. 7 includes a pair of enlarged extension portions 31 provided at the opposite ends of the net body 15. Each of the enlarged extension portions 33 is tapered such that the diameter thereof become gradually larger away from the end of the net body 15. The enlarged extension portions 33 serve to assure smooth flow of the bodily fluid and to increase the fixing force of the stent 31. In case of the stent 31 shown in Fig. 8, each of the enlarged extension portions 32 is of a sleeve shape and has a diameter greater than that of the net body 15.

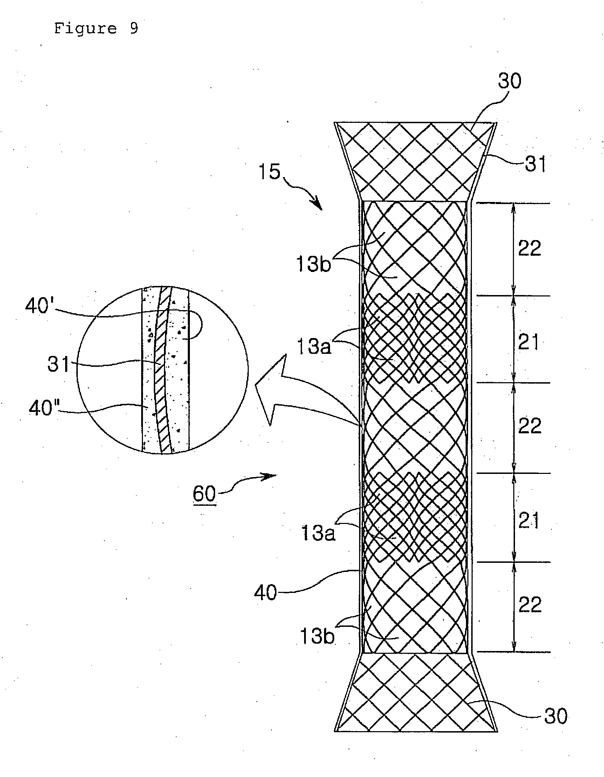

The stent 60 shown in Fig. 9 includes a pair of enlarged extension portions 31 provided at the opposite ends of the net body 15 and a resin film layer 40 formed on the net body 15 and enlarged extension portions 31. Each of the enlarged extension portions 31 has a tapering shape.

The resin film layer 40 is made of, e.g., polytetrafluoroethylene (PTFE) and silicon. The resin film layer 40 may be divided into a first resin film layer 40' formed on the inner circumferential surface of the net body 15 and the enlarged extension portions 31 and a second resin film layer 40" formed on the outer circumferential surface of the net body 15 and the enlarged extension portions 31.

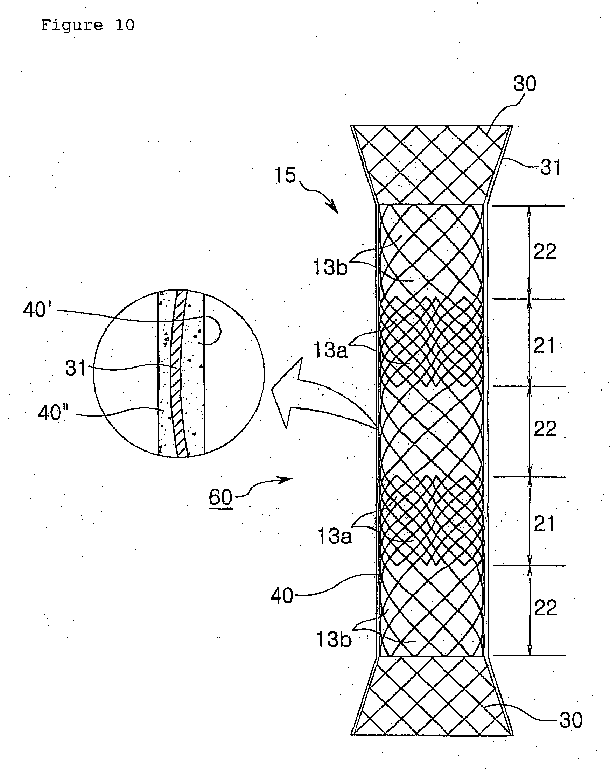

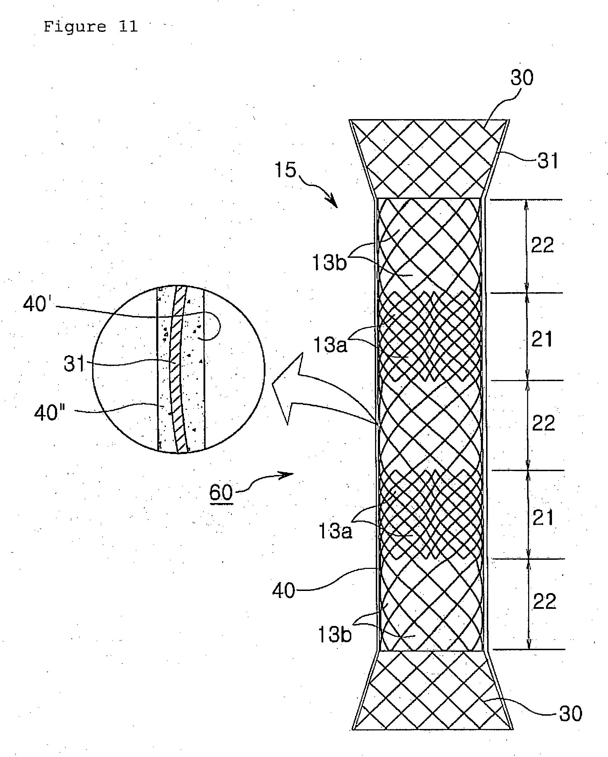

In case of the stent 60 shown in Fig. 10, the first resin film layer 40' and the second resin film layer 40'' are made of different resins. More specifically, the first resin film layer 40' is made of silicon and the second resin film layer 40" is made of polytetrafluoroethylene. In case of the stent 60 shown in Fig. 11, the first resin film layer 40' is made of polytetrafluoroethylene and the second resin film layer 40" is made of silicon.

The first and second resin film layers 40' and 40" thus formed serve mainly to prevent the stenosed part of the bodily organ from growing into the stent 60 through the meshes 13a and 13b of the net body 15. If the first and second resin film layers 40' and 40" are made of different resins as in Figs. 10 and 11, it is possible to enhance the resistance of the resin film layers 40' and 40'to the bodily fluid, thereby allowing the stent 60 to perform its function for a prolonged period of time.

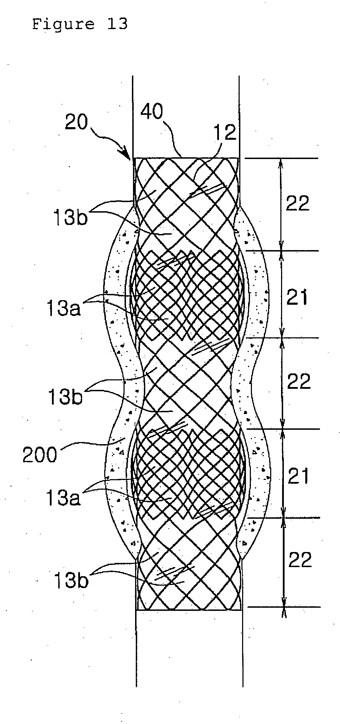

Use and operation of the wavily deformable stent 20 will be described with reference to Fig. 13.

As shown in Fig. 13, the stent 20 is situated inside the stenosed part of a bile duct or other bodily organs to expand the same radially outwards. It is preferred that the stent 20 used for this purpose has a length equal to or a little greater than the length of the stenosed part.

Since the net body 15 of the stent 20 includes the high-rigidity sections 21 and the low-rigidity sections 22 as set forth above, the stent 20 is deformed into a wavelike form when situated inside the stenosed part. In other words, the high-rigidity sections 21 expand the stenosed part with a greater expansion force but the low-rigidity sections 22 expand the stenosed part with an expansion force smaller than that of the high-rigidity sections 21.

This helps increase the frictional contact force acting between the stent 20 and the stenosed part, thereby reducing the tendency of sliding movement of the stent 20 within the stenosed part. Therefore, it is possible to prevent the stent 20 from being displaced out of the stenosed part during its use.

While certain embodiments of the present invention have been described hereinabove, the present invention shall not be limited thereto. It will be understood by those skilled in the art that various changes and modifications may be made without departing from the scope of the invention defined in the claims.

## Claims

1. A wavily deformable stent comprising a hollow cylindrical net body (15) formed of elastically deformable wires (12) interlaced with each other, the net body (15) extending in a longitudinal direction and terminating at open opposite ends, the net body (15) including at least one high-rigidity section (21) and at least one low-rigidity section (22) less rigid than the high-rigidity section (21), **characterized in that**:
the or each high-rigidity section (21) and the or each low-rigidity section (22) are arranged continuously and alternately along the longitudinal direction;
the at least one high-rigidity section (21) is formed of crossed and bent interlaced wires (12), the wires (12) being interlaced at a first, narrow interval; and
the at least one low-rigidity section (22) is also formed of crossed and bent interlaced wires (12), the wires (12) being interlaced at a second, broader interval.

2. A stent as recited in claim 1, wherein the high-rigidity section (21) has a plurality of first meshes (13a) and the low-rigidity section (22) has a plurality of second meshes (13b) greater in average size and smaller in number than the first meshes (13a).

3. A stent as recited in claim 1 or 2, wherein the high-rigidity section (21) and the low-rigidity section (22) differ in length from each other.

4. A stent as recited in any preceding claim, further comprising a pair of enlarged extension portions (30) provided at the opposite ends of the net body (15), the enlarged extension portions (30) being greater in diameter than the net body (15).

5. A stent as recited in any preceding claim, further comprising a resin film layer (40) formed on the net body (15) and, if present, the enlarged extension portions (30).

6. A stent as recited in claim 5, wherein the resin film layer (40) is made of a substance selected from the group consisting of polytetrafluoroethylene and silicon.

7. A stent as recited in any of claims 1 to 4, wherein the net body (15) has an inner circumferential surface and an outer circumferential surface, and further comprising a first resin film layer (40) formed on the inner circumferential surface of the net body and a second resin film layer (40") formed on the outer circumferential surface of the net body (15).

8. A stent as recited in claim 7, wherein the first resin film layer (40) and the second resin film (40") layer are made of different resins.

9. A stent as recited in any preceding claim and having two high-rigidity sections (21) and three low-rigidity sections (22).

10. A method for producing a wavily deformable stent, comprising the steps of:
- preparing elastically deformable wires (12); and
- interlacing the wires (12) with each other by crossing and bending to form a hollow cylindrical net body (15) having at least one high-rigidity section (21) and at least one low-rigidity section (22) less rigid than the high-rigidity section (21), the net body extending in a longitudinal direction and terminating at open opposite ends, wherein the or each high-rigidity section (21) and the or each low-rigidity section (22) are arranged continuously and alternately along the longitudinal direction;
wherein the at least one high-rigidity section (21) is formed by crossing and bending the interlaced wires (12) at a first, narrow interval; and
the at least one low-rigidity section (22) is formed by crossing and bending the interlaced wires (12) at a second, broader interval.

11. A method as recited in claim 10, further comprising the step of: providing a pair of enlarged extension portions (30) at the opposite ends of the net body (15), the enlarged extension portions (30) being greater in diameter than the net body (15).

12. A method as recited in claim 10 or 11, further comprising the step of: forming a resin film layer (40) on the net body (15).

13. A method as recited in any of claims 10 to 12, wherein the wires (12) are interlaced by crossing and bending at pins (120) of a jig (100).

## Patentansprüche

1. Wellenförmig verformbarer Stent, umfassend einen hohlen zylinderförmigen Netzkörper (15), der aus elastisch verformbaren Drähten (12), die miteinander verflochten sind, ausgebildet ist, wobei der Netzkörper (15) sich in einer Längsrichtung erstreckt und in gegenüberliegenden offenen Enden endet, wobei der Netzkörper (15) mindestens einen Bereich hoher Steifigkeit (21) und mindestens einen Bereich niedriger Steifigkeit (22), der weniger starr ist als der Bereich hoher Steifigkeit (21), aufweist, **dadurch gekennzeichnet, dass**:
der oder jeder Bereich hoher Steifigkeit (21) und der oder jeder Bereich niedriger Steifigkeit (22) kontinuierlich und abwechselnd entlang der Längsrichtung angeordnet sind;
der mindestens eine Bereich hoher Steifigkeit (21) aus gekreuzt und gebogen verflochtenen Drähten (12) ausgebildet ist, wobei die Drähte (12) mit einem ersten schmalen Zwischenraum verflochten sind; und
der mindestens eine Bereich niedriger Steifigkeit (22) auch aus gekreuzt und gebogen verflochtenen Drähten (12) ausgebildet ist, wobei die Drähte (12) mit einem zweiten breiteren Zwischenraum verflochten sind.

2. Stent nach Anspruch 1, wobei der Bereich hoher Steifigkeit (21) eine Vielzahl erster Maschen (13a) aufweist und der Bereich niedriger Steifigkeit (22) eine Vielzahl zweiter Maschen (13b) aufweist, die in der Durchschnittsgröße größer und in der Anzahl geringer als die ersten Maschen (13a) sind.

3. Stent nach Anspruch 1 oder 2, wobei der Bereich hoher Steifigkeit (21) und der Bereich niedriger Steifigkeit (22) sich in der Länge voneinander unterscheiden.

4. Stent nach einem der vorhergehenden Ansprüche, ferner umfassend ein Paar von vergrößerten Verlängerungsabschnitten (30), die an den entgegengesetzten Enden des Netzkörpers (15) vorgesehen sind, wobei die vergrößerten Verlängerungsabschnitte (30) im Durchmesser größer sind als der Netzkörper (15).

5. Stent nach einem der vorhergehenden Ansprüche, ferner umfassend eine Harzfilmschicht (40), die auf dem Netzkörper (15) und, falls vorhanden, auf den vergrößerten Verlängerungsabschnitten (30) ausgebildet ist.

6. Stent nach Anspruch 5, wobei die Harzfilmschicht (40) aus einer Substanz hergestellt ist, die ausgewählt ist aus der Gruppe bestehend aus Polytetrafluorethylen und Silikon.

7. Stent nach einem der Ansprüche 1 bis 4, wobei der Netzkörper (15) eine innere Umfangsfläche und eine äußere Umfangsfläche aufweist und ferner eine erste Harzfilmschicht (40), die auf der inneren Umfangsfläche des Netzkörpers ausgebildet ist, und eine zweite Harzfilmschicht (40") aufweist, die auf der äußeren Umfangsfläche des Netzkörpers (15) ausgebildet ist.

8. Stent nach Anspruch 7, wobei die erste Harzfilmschicht (40) und die zweite Harzfilmschicht (40") aus unterschiedlichen Harzen hergestellt sind.

9. Stent nach einem der vorhergehenden Ansprüche und mit zwei Bereichen hoher Steifigkeit (21) und drei Bereichen niedriger Steifigkeit (22).

10. Verfahren zur Herstellung eines wellenförmig verformbaren Stents, umfassend die Schritte:
- Vorbereiten elastisch verformbarer Drähte (12);
und
- Verflechten der Drähte (12) miteinander durch Kreuzen und Biegen, um einen hohlen zylinderförmigen Netzkörper (15) auszubilden, der mindestens einen Bereich hoher Steifigkeit (21) und mindestens einen Bereich niedriger Steifigkeit (22) aufweist, der weniger starr ist als der Bereich hoher Steifigkeit (21), wobei der Netzkörper sich in einer Längsrichtung erstreckt und in offenen gegenüberliegenden Enden endet und wobei der oder jeder Bereich hoher Steifigkeit (21) und der oder jeder Bereich niedriger Steifigkeit (22) kontinuierlich und abwechselnd entlang der Längsrichtung angeordnet sind;
wobei der mindestens eine Bereich hoher Steifigkeit (21) durch Kreuzen und Biegen der verflochtenen Drähte (12) mit einem ersten schmalen Zwischenraum gebildet wird; und
der mindestens eine Bereich niedriger Steifigkeit (22) durch Kreuzen und Biegen der verflochtenen Drähte (12) mit einem zweiten breiteren Zwischenraum gebildet wird.

11. Verfahren nach Anspruch 10, ferner umfassend den Schritt: Bereitstellen eines Paares vergrößerter Verlängerungsabschnitte (30) an den entgegengesetzten Enden des Netzkörpers (15), wobei die vergrößerten Verlängerungsabschnitte (30) im Durchmesser größer sind als der Netzkörper (15).

12. Verfahren nach Anspruch 10 oder 11, ferner umfassend den Schritt: Ausbilden einer Harzfilmschicht (40) auf dem Netzkörper (15).

13. Verfahren nach einem der Ansprüche 10 bis 12, wobei die Drähte (12) durch Kreuzen und Biegen an Stiften (120) einer Montagevorrichtung (100) verflochten werden.

## Revendications

1. Endoprothèse déformable de manière ondulée comprenant un corps net cylindrique creux (15) réalisé par des fils élastiquement déformables (12) entrelacés les uns avec les autres, le corps net (15) s'étendant dans une direction longitudinal et se terminant à des extrémités opposées ouvertes, le corps net (15) incluant au moins une section de rigidité élevée (21) et au moins une section de rigidité basse (22) moins rigide que la section de rigidité élevée (21), **caractérisée en ce que** :
la ou chaque section de rigidité élevée (21) et la ou chaque section de rigidité basse (22) sont agencées continuellement et alternativement le long de la direction longitudinale ;
la au moins une section de rigidité élevée (21) est réalisée par des fils entrelacés croisés et courbés (12), les fils (12) étant entrelacés selon un premier intervalle étroit ; et
la au moins une section de rigidité basse (22) est également réalisée par des fils entrelacés croisés et courbés (12), les fils (12) étant entrelacés selon un deuxième intervalle plus large.

2. Endoprothèse selon la revendication 1, dans laquelle la section de rigidité élevée (21) comporte une pluralité de premières mailles (13a), et la section de rigidité basse (22) comporte une pluralité de deuxièmes mailles (13b) plus grandes en taille moyenne et plus petites en nombre que les premières mailles (13a).

3. Endoprothèse selon la revendication 1 ou 2, dans laquelle la section de rigidité élevée (21) et la section de rigidité basse (22) diffèrent en longueur l'une de l'autre.

4. Endoprothèse selon l'une quelconque des revendications précédentes, comprenant en outre une paire de portions d'extension élargies (30) réalisées aux extrémités opposées du corps net (15), les portions d'extension élargies (30) ayant un diamètre plus grand que le corps net (15).

5. Endoprothèse selon l'une quelconque des revendications précédentes, comprenant en outre une couche de film de résine (40) formée sur le corps net (15) et, si présentes, les portions d'extension élargies (30).

6. Endoprothèse selon la revendication 5, dans laquelle la couche de film de résine (40) est réalisée en une substance sélectionnée dans le groupe consistant en polytétrafluoroéthylène et silicium.

7. Endoprothèse selon l'une quelconque des revendications 1 à 4, dans laquelle le corps net (15) comporte une surface circonférentielle intérieure et une surface circonférentielle extérieure, et comprenant en outre une première couche de film de résine (40) formée sur la surface circonférentielle intérieure du corps net et une deuxième couche de film de résine (40") formée sur la surface circonférentielle extérieure du corps net (15).

8. Endoprothèse selon la revendication 7, dans laquelle la première couche de film de résine (40) et la deuxième couche de film de résine (40") sont réalisées en des résines différentes.

9. Endoprothèse selon l'une quelconque des revendications précédentes et ayant deux sections de rigidité élevée (21) et trois sections de rigidité basse (22).

10. Procédé de production d'une endoprothèse déformable de manière ondulée, comprenant les étapes de :
- préparer des fils élastiquement déformables (12) ; et
- entrelacer les fils (12) les uns avec les autres par croisement et courbure pour former un corps net cylindrique creux (15) ayant au moins une section de rigidité élevée (21) et au moins une section de rigidité basse (22) moins rigide que la section de rigidité élevée (21), le corps net s'étendant dans une direction longitudinale et se terminant aux extrémités opposées ouvertes, où la ou chaque section de rigidité élevée (21) et la ou chaque section de rigidité basse (22) sont agencées de manière continue et alternante dans la direction longitudinale ;
où la au moins une section de rigidité élevée (21) est formée en croisant et en courbant les fils entrelacés (12) selon un premier intervalle étroit ; et
la au moins une section de rigidité basse (22) est formée en croisant et en courbant les fils entrelacés (12) selon un deuxième intervalle plus large.

11. Procédé selon la revendication 10, comprenant en outre l'étape de : réaliser une paire de portions d'extension élargies (30) aux extrémités opposées du corps net (15), les portions d'extension élargies (30) ayant un diamètre plus grand que le corps net (15).

12. Procédé selon la revendication 10 ou 11, comprenant en outre l'étape de : former une couche de film de résine (40) sur le corps net (15).

13. Procédé selon l'une quelconque des revendications 10 à 12, dans lequel les fils (12) sont entrelacés par croisement et courbure à des broches (120) d'un gabarit (100).
